# EUROPÄISCHE PATENTANMELDUNG

(11) **EP 0 001 424 A1**
(43) Veröffentlichungstag der Anmeldung: **18.04.1979**
(21) Anmeldenummer: 78100979.0
(22) Anmeldetag: 25.09.1978
(51) Int. Cl.: A01N 43/16, C07D 311/96, C07D 311/22

(54) **Insektizide Mittel**

(30) Priorität: 07.10.1977 DE 2745306
(71) Anmelder: BAYER AG, 51368 Leverkusen (DE)
(72) Erfinder: Kabbe, Hans-Joachim, Dr., D-5090 Leverkusen 1 (DE); Roessler, Peter, Dr., D-5060 Bergisch Gladbach (DE)

(57) **Zusammenfassung**

Die vorliegende Erfindung betrifft die Verwendung von Chromanonen-(4) der allgemeinen Formel (1) 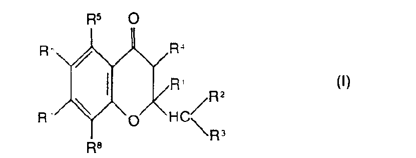 worin R-R⁸ die in der Beschreibung angegebene Bedeutung haben, als Insektizide und Akarizide.

## Beschreibung

Die vorliegende Erfindung betrifft die Verwendung von bekannten Chromanonen-(4) zur Bekämpfung von Insekten und Spinnentieren.

Es ist bereits bekannt geworden, daß Chromanone herbizide und antioxidative Eigenschaften besitzen (vgl. Deutsche Offenlegungsschrift 2 535 338).

Außerdem ist bekannt geworden, daß bestimmte Chromene, z.B. Precocen I und Precocen II entwicklungsinhibierende Eigenschaften aufweisen (Chem. Eng. News 1976, Heft 16, Seite 19). Ihre Wirkung ist jedoch, vor allem bei niedrigen Aufwandmengen, nicht voll befriedigend.

Es wurde nun gefunden, daß die bekannten Chromanone-(4) der allgemeinen Formel (I) 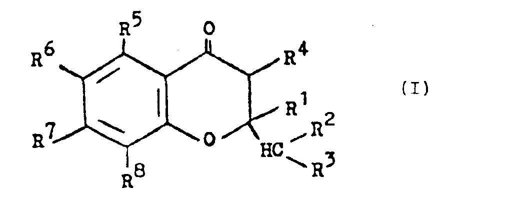 worin
R¹ bis _{R}⁴ gleich oder verschieden sind und für Wasserstoff, gegebenenfalls substituiertes Alkyl, Alkenyl, Cycloalkyl, Aryl, Aralkyl, Alkoxycarbonyl, Carboxyl oder Aminoalkyl stehen , .
_{R}² zusätzlich für _{A}mino und Dialkylamino stehen kann und
R¹ und R² gemeinsam mit den angrenzenden C-Atomen einen carbocyclischen oder heterocyclischen Ring bilden können und
_{R}⁵ bis _{R}⁸ gleich oder verschieden sind und für Wasserstoff, Halogen, Hydroxy, Nitro, Cyan, Carboxy, gegebenenfalls substituiertes Alkyl, Alkenyl, Cycloalkyl, Aryl, Aralkyl, Alkoxy, Aralkoxy, Aryloxy, Alkoxycarbonyl, Amino, Alkylamino, Dialkylamino oder Acylamino stehen,

starke insektizide und akarizide Eigenschaften aufweisen.

Überraschenderweise zeigen die erfindungsgemäßen Wirkstoffe eine erheblich bessere Wirkung als die aus dem Stand der Technik bekannten Verbindungen ähnlicher Struktur und analoger Wirkung. Die neue Verwendung der Wirkstoffe stellt somit eine Bereicherung der Technik dar.

Die erfindungsgemäßen Wirkstoffe der allgemeinen Formel (I) und ihre Herstellung sind bekannt aus der Deutschen Offenlegungsschrift 2 535 338.

In der allgemeinen Formel (I) seien als gegebenenfalls substituierte Alkyl- oder Alkenylreste (R bis R⁸) geradkettige oder verzweigte Alkyl- oder Alkenylreste mit bis zu 18, bevorzugt bis zu 12, besonders bevorzugt bis zu 6 Kohlenstoffatomen genannt. Als solche seien beispielsweise genannt: Methyl, Äthyl, Propyl, Isopropyl, Butyl, Isobutyl, tert.-Butyl, Hexyl, Nonyl, Decyl, Undecyl, Octadecyl, Buten-(3)-yl, 4-Methyl-penten-(3)-yl, 4,8-Dimethylnonadien-(3,7)-yl.

Als gegebenenfalls substituierte Cycloalkylreste (R bis R°) kommen beispielsweise solche mit 3 bis 18, bevorzugt mit 4 bis 12, besonders bevorzugt mit 5 und 6 Kohlenstoffatomen, wie beispielsweise Cyclopropyl, Cyclobutyl, Cyclopentyl, Cyclohexyl, Cycloheptyl, Cyclooctyl, Cyclododecyl, Cycloheptadecyl und Cyclooctadecyl, bevorzugt Cyclopentyl und Cyclohexyl, in Frage.

Als gegebenenfalls substituierte Arylreste (R bis R°) seien solche mit 6 bis 14 Kohlenstoffatomen genannt, wie Phenyl, Naphthyl, Anthracyl, bevorzugt Phenyl.

Als gegebenenfalls substituierte Aralkylreste (R bis R⁸) kommen beispielsweise solche mit 7 bis 18 Kohlenstoffatomen, deren aliphatischer Teil 1 bis 8, bevorzugt 1 bis 4, Kohlenstoffe enthält und deren aromatischer Teil einen carbocyclischen Rest mit 6 bis 10 Kohlenstoffatomen darstellt, in Frage. Beispielsweise seien die folgenden Aralkylreste genannt: Benzyl, Phenyläthyl, Phenylpropyl, Phenylbutyl, Naphthylmethyl und Naphthyläthyl, bevorzugt Benzyl.

Wenn R¹ mit R² unter Ausbildung eines gegebenenfalls substituierten Ringes verbunden ist, kann dieser sowohl carbocyclisch als auch heterocyclisch sein.

Als carbocyclische Ringe (R /R ) kommen beispielsweise gesättigte oder ungesättigte Kohlenwasserstoffglieder enthaltende Ringe, bevorzugt 3- bis 12-gliedrige Ringe, in Frage. Es ist auch möglich, daß die carbocyclischen Ringe an einen oder mehrere Reste aus der Benzolreihe annelliert sind.

Als carbocyclische Reste seien beispielsweise genannt: Cyclopropan, Cyclopentan, Cyclohexan, Cycloheptan, Cyclooctan, Cyclononan, Cyclodecan, Cycloundecan, Cyclododecan, Cyclohexen, Cycloocten, Cyclododecen und Tetralin.

Als heterocyclische Ringe (R¹/R²) kommen beispielsweise 5- bis 12-gliedrige Ringe, bevorzugt 5- und 6-gliedrige Ringe in Frage, die außer Kohlenwasserstoffgliedern noch ein oder mehrere Heteroatome, wie beispielsweise Stickstoff, Sauerstoff und/oder Schwefel enthalten. Die heterocyclischen Ringe können 1 oder 2 Doppelbindungen enthalten und außerdem an einen oder mehrere Reste aus der Benzolreihe anelliert sein. Als heterocyclische Reste seien beispielsweise genannt: Piperidin, Pyrrolidin, Tetrahydrofuran, Tetrahydropyran und Tetrahydrothiopyran.

Die Alkyl- bzw. Arylreste der Alkoxy-, Alkoxycarbonyl-, Alkylamino-, Dialkylamino-, Aryloxy- und der Aralkoxyreste entsprechen hinsichtlich ihrer Kohlenstoffzahl dem vorstehend angegebenen Bedeutungsumfang.

Als bevorzugte Alkoxygruppen seien solche mit bis zu 4 Kohlenstoffatomen, wie Methoxy, Äthoxy, Propoxy, Isopropoxy, Butoxy, Isobutoxy und tert.-Butoxy, genannt.

Als bevorzugte Aryloxygruppen seien solche mit 6 oder 10 Kohlenstoffatomen, wie Phenoxy oder Naphthoxy, genannt.

Als bevorzugte Aralkoxygruppen seien solche mit 7 bis 10 Kohlenstoffatomen, wie Benzyloxy, Phenyläthoxy, Phenylpropoxy, Phenylisopropoxy, Phenylbutoxy, Phenylisobutoxy und Phenyl-tert.-butoxy, genannt.

Als bevorzugte Alkoxycarbonylgruppen seien solche mit bis zu 4 Kohlenstoffatomen im Alkylrest, wie Methoxycarbonyl, Äthoxycarbonyl, Propoxycarbonyl, Isopropoxycarbonyl, genannt.

Als bevorzugte Alkyl- und Dialkylaminogruppen seien solche mit bis zu 3 Kohlenstoffatomen im Alkylrest, wie Methylamino, Äthylamino, Isopropylamino, Dimethylamino, Diäthylamino, Dipropylamim und _{D}iisopropylamino, genannt. Es ist auch möglich, daß die beiden Alkylreste der Dialkylaminogruppe zu einem Ring geschlossen sind, wie beispielsweise Pyrrolidinyl, Piperidinyl.

Die Acylaminogruppe (R⁵ bis R⁸) kann durch einen aliphatischen oder aromatischen Rest substituiert sein, wobei der aliphatische und der aromatische Rest den oben genannten Bedeutungsumfang haben. Als Acylaminogruppen seien

beispielsweise genannt: Formylamino, Acetylamino, Propionylamino, Valeroylamino und Benzoylamino.

Als Halogene seien Fluor, Chlor, Brom und Jod, bevorzugt Chlor, genannt.

Als Substituenten der Alkyl-, Cycloalkyl-, Aryl-, Aralkyl-, Alkoxy-, Aralkoxy-, Alkoxycarbonyl-, Alkylamino- und Dialkylaminogruppen der Reste R¹ bis R⁸ kommen Substituenten in Frage, die unter den Reaktionsbedingungen nicht verändert werden. Beispielsweise seien die Halogene, wie Fluor, Chlor, Brom und Jod, die Cyangruppe, C₁-c₆-Alkylgruppe, die C₁-C₆-Alkoxygruppe, die C₁-C₆-Alkoxycarbonylgruppe, die c₁-c₆-Alkoxycarbonylalkylgruppe, die Aminogruppe, die C₁-C₆-Alkylamino- und die C1-C6-Dialkylaminogruppe, Arylreste aus der Benzolreihe oder die Carbonsäuregruppe genannt.

Als Beispiele für die erfindungsgemäß verwendbaren Chromanone-(4) der Formel (I) seien beispielsweise genannt:
2,2-Pentamethylenchromanon-(4)
2,2-Pentamethylen-7-hydroxychromanon-(4)
2,2-Pentamethylen-6-hydroxychromanon-(4)
2,2-Pentamethylen-6-methoxychromanon-(4)
2,2-Pentamethylen-7-methoxychromanon-(4)
7-Acetylamino-2,2-pentamethylenchromanon-(4)
6-Cyclohexyl-2,2-pentamethylenchromanon-(4)
5-Chlor-7-phenyl-2,2-pentamethylenchromanon-(4)
7-Alkyloxy-2,2-pentamethylenchromanon-(4)
6-Äthoxycarbonylmethoxy-2,2-pentamethylenchromanon-(4)
6-Nitro-2,2-pentamethylenchromanon-(4)
5-Cyan-2,2-pentamethylenchromanon-(4)
7-Trifluormethyl-2,2-pentamethylenchromanon-(4)
6-Carboxy-2,2-pentamethylenchromanon-(4)
7-Methoxycarbonyl-2,2-pentamethylenchromanon-(4)
6-Butyramido-2,2-pentamethylenchromanon-(4)
7-Amino-2,2-pentamethylenchromanon-(4)
5-Hydroxy-7-pentyl-2,2-pentamethylenchromanon-(4)
2-Methyl-2-( 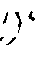 )-diäthylaminopropyl)-chromanon-(4)
2-Methyl-2-(B-carboxyäthyl)-chromanon-(4)
2-Methyl-2-nonyl-7-hydroxy-chromanon-(4)
2-Methyl-2-(B-N-pyrrolidinyläthyl)-chromanon-(4)
2-Methyl-2-( 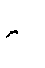 -carboxybutyl)-chromanon-(4)
2,2-Tetramethylenchromanon-(4)
7-Hydroxy-2,2-tetramethylenchromanon-(4)
6-Hydroxy-2,2-tetramethylenchromanon-(4)
8-Methoxy-2,2-tetramethylenchromanon-(4)
6-Äthoxy-2,2-tetramethylenchromanon-(4)
7-Chlor-2,2-tetramethylenchromanon-(4)
5-Brom-2,2-tetramethylenchromanon-(4)
2-Isopropyl-3-phenyl-6-methyl-chromanon-(4)
2,3,6-Trimethyl-chromanon-(4)
5,7-Dihydroxy-2,2-tetramethylenchromanon-(4)
6,8-Dihydroxy-2,2-tetramethylenchromanon-(4)
5,8-Dihydroxy-2,2-tetramethylenchromanon-(4)
5,7,8-Trihydroxy-2,2-tetramethylenchromanon-(4)
7-Benzyloxy-2,2-tetramethylenchromanon-(4)
6-Dimethylamino-2-isopropyl-chromanon-(4)
7-Acetamino-2-isopropyl-chromanon-(4)
7-Chlor-2-propyl-chromanon-(4)
6-Hexylamino-2-methyl-2-nonyl-chromanon-(4)
5-Hydroxy-7-pentyl-2,2-tetramethylen-chromanon-(4)
5-Hydroxy-7-pentyl-2,2-undecamethylen-chromanon-(4)
5-Hydroxy-7-heptyl-2-methyl-2-nonyl-chromanon-(4)
5-Methyl-7-hydroxy-2-methyl-2-o-carboxybutyl-chromanon-(4)
6-Hydroxy-2-methyl-2-6 -carboxybutyl-chromanon-(4)
7-Hydroxy-2- δ -carboxybutyl-chromanon-(4)
5-Hydroxy-7-pentyl-2-methyl-2-ß-carboxyäthyl-chromanon-(4)
6-Hydroxy-2-methyl-2-ß,ß,ß-trifluoräthyl-chromanon-(4)
7-Hydroxy-2-methyl-2-diäthylaminopropyl-chromanon-(4)
2-Methyl-2-N-pyrrolidinylpropyl-chromanon-(4)
2-Methyl-2-benzyl-chromanon-(4)
2-Hydroxybutyl-chromanon-(4)

Die Wirkstoffe eignen sich bei guter Pflanzenverträglichkeit und günstiger Warmblütertoxizität zur Bekämpfung von tierischen Schädlingen, insbesondere Insekten und Spinnentieren, die in der Landwirtschaft, in Forsten, im Vorrats- und Materialschutz sowie auf dem Hygienesektor vorkommen. Sie sind gegen normal sensible und resistente Arten sowie gegen alle oder einzelne Entwicklungsstadien wirksam. Zu den oben erwähnten Schädlingen gehören:
Aus der Ordnung der Isopoda z. B. Oniscus asellus, Armadillidium vulgare, Porcellio scaber.
Aus der Ordnung der Diplopoda z. B. Blaniulus guttulatus.
Aus der Ordnung der Chilopoda z. B. Geophilus carpophagus, Scutigera spec.
Aus der Ordnung der Symphyla z. B. Scutigerella immaculata.
Aus der Ordnung der Thysanura z. B. Lepisma saccharina.
Aus der Ordnung der Collembola z. B. Onychiurus armatus.
Aus der Ordnung der Orthoptera z. B. Blatta orientalis, Periplaneta americana, Leucophaea maderae, Blattella germanica, Acheta domesticus, Gryllotalpa spp., Locusta migratoria migratorioides, Melanoplus differentialis, Schistocerca gregaria.
Aus der Ordnung der Dermaptera z. B. Forficula auricularia.
Aus der Ordnung der Isoptera z. B. Reticulitermes spp..
Aus der Ordnung der Anoplura z. B. Phylloxera vastatrix, Pemphigus spp., Pediculus humanus corporis, Haematopinus spp., Linognathus spp.
Aus der Ordnung der Mallophaga z.B. Trichodectes spp., Damalinea spp.
Aus der Ordnung der Thysanoptera z.B. Hercinothrips femoralis, Thrips tabaci.
Aus der Ordnung der Heteroptera z.B. Eurygaster spp., Dysdercus intermedius, Piesma auadrata, Cimex lectularius, Rhodnius prolixus, Triatoma spp.
Aus der Ordnung der Homoptera z.B. Aleurodes brassicae, _{B}emisia tabaci, Trialeurodes vaporariorum, Aphis gossypii, Brevicoryne brassicae, Cryptomyzus ribis, Doralis fabae, Doralis pomi, Erjosoma lanigerum, Hyalopterus arundinis, Macrosiphum avenae, Myzus spp., Phorodon humuli, Rhopalosiphum padi, Empoasca spp., Euscelis bilobatus, Nephotettix cincticeps, Lecanium corni, Saissetia oleae, Laodelphax striatellus, Nilaparvata lugens, Aonidiella aurantii, Aspidiotus hederae, Pseudococcus spp., Psylla spp..
Aus der Ordnung der Lepidoptera z. B. Pectinophora gossypiella, Bupalus piniarius, Cheimatobia brumata, Lithocolletis blancardella, Hyponomeuta padella, Plutella maculipennis, Malacosoma neustria, Euproctis chrysorrhoea, Lymantria spp., Bucculatrix thurberiella, Phyllocnistis citrella, Agrotis spp., Euxoa spp., Feltia spp., Earias insulana, Heliothis spp., Laphygma exigua, Mamestra brassicae, Panolis flammea, Prodenia litura, Spodoptera spp., Trichoplusia ni, Carpocapsa pomonella, Pieris spp., Chilo spp., Pyrausta nubilalis, Ephestia kuehniella, Galleria mellonella, Cacoecia podana, Capua reticulana, Choristoneura fumiferana, Clysia ambiguella, Homona magnanima, Tortrix viridana.
Aus der Ordnung der Coleoptera z. B. Anobium punctatum, Rhizopertha dominica, Bruchidius obtectus, Acanthoscelides obtectus, Hylotrupes bajulus, Agelastica alni, Leptinotarsa decemlineata, Phaedon cochleariae, Diabrotica spp., Psylliodes chrysocephala, Epilachna varivestis, Atomaria sop., Oryzaephilus surinamensis, Anthonomus spp., Sitophilus spp., Otiorrhynchus sulcatus, Cosmopolites sordidus, Ceuthorrhynchus assimilis, Hypera postica, Dermestes spp., Trogoderma spp., Anthrenus spp., Attagenus spp., Lyctus spp., Meligethes aeneus, Ptinus spp., Niptus hololeucus, Gibbium psylloides, Tribolium spp., Tenebrio molitor, Agriotes spp., Conoderus spp., Melolontha melolontha, Amphimallon solstitialis, Costelytra zealandica.
Aus der Ordnung der Hymenoptera z. B. Diprion spp., Hoplocampa spp., Lasius spp., Monomorium pharaonis, Vespa spp.
Aus der Ordnung der Diptera z.B. Aedes spp., Anopheles sop., Culex spp., Drosophila melanogaster, Musca spp., Fannia spp., Calliphora erythrocephala, Lucilia spp., Chrysomyia spp., Cuterebra spp., Gastrophilus s^{p}p., Hyppobosca spn., Stomoxys spp., Oestrus spp., Hypoderma spp., Tabanus sDp., Tannia spp., Bibio hortulanus, Oscinella frit, Phorbia snp., Pegomyia hyoscyami, Ceratitis capitata, Dacus oleae, Tipula paludosa.
Aus der Ordnung der Siphonantera z.B. Xenopsylla cheopis, Ceratophyllus spp..
Aus der Ordnung der Arachnida z.B. Scorpio maurus, Latrodectus mactans.
Aus der Ordnung der Acarina z.B. Acarus siro, Argas spn., Ornithodoros spp., Dermanyssus gallinae, Eriophyes ribis, Phyllocoptruta oleivora, Boophilus spp., Rhipicephalus spo., Amblyomma spp., Hyalomma spp., Ixodes spp., Psoroptes sop., Chorioptes spp., Sarcoptes spp., Tarsonemus sp^{p.}, Bryobia praetiosa, Panonychus spp., Tetranychus spo..

Die Wirkstoffe können in die üblichen Formulierungen übergeführt werden, wie Lösungen, Emulsionen, Spritzpulver, Suspensionen, Pulver, Stäubemittel, Schäume, Pasten, lösliche Pulver, Granulate, Aerosole, Suspensions-Emulslonskonzentrate, Saatgutpuder, Wirkstoff-imprägnierte Natur- und synthetische Stoffe, Feinstverkapselungen in polymeren Stoffen und in Hüllmassen für Saatgut, ferner in Formulierungen mit Brennsätzen, wie Räucherpatronen, -dosen, -spiralen u.ä. sowie ULV-Kalt- und Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen infrage: Aromaten, wie Xylol, Toluol, oder Alkylnaphthaline, chlorierte Aromaten oder chlorierte aliphatische Kohlenwasserstoffe, wie Chlorbenzole, Chloräthylene oder Methylenchlorid, aliphatische Kohlenwasserstoffe, wie Cyclohexan oder Paraffine, z.B. Erdölfraktionen, Alkohole, wie Butanol oder Glycol sowie deren Äther und Ester, Ketone, wie Aceton, Methyläthylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgas, wie Halogenkohlenwasserstoffe sowie Butan, Propan, Stickstoff und Kohlendioxid; als feste Trägerstoffe: natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate: gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehle, Kokosnußschalen, Maiskolben und Tabakstengel; als Emulgier-und/oder schaumerzeugende Mittel: nichtionogene und anionische Emulgatoren, wie Polyoxyäthylen-Fettsäure-Ester, Polyoxyäthylen-Fettalkohol-Äther, z.B. Alkylaryl-polyglykol-äther, Alkylsulfonate, Alkylsulfate, Arylsulfonate sowie Eiweißhydrolysate; als Dispergiermittel: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat.

Es können Farbstoffe wie anorganische Pigmente, z.B. Eisenoxid, Titanoxid, Ferrocyanblau und organische Farbstoffe, wie Alizarin-, Azo-Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90%.

Die Anwendung der erfindungsgemäßen Wirkstoffe erfolgt in Form ihrer handelsüblichen Formulierungen und/oder den aus diesen Formulierungen bereiteten Anwendungsformen.

Der Wirkstoffgehalt der aus den handelsüblichen Formulierungen bereiteten Anwendungsformen kann in weiten Bereichen variieren. Die Wirkstoffkonzentration der Anwendungsformen kann von 0,0000001 bis zu 100 Gew.-% Wirkstoff, vorzugsweise zwischen 0,01 und 10 Gew.-% liegen.

Die Anwendung geschieht in einer den Anwendungsformen angepaßten üblichen Weise.

Bei der Anwendung gegen Hygiene- und Vorratsschädlinge zeichnen sich die Wirkstoffe durch eine hervorragende Residualwirkung auf Holz und Ton sowie durch eine gute Alkalistabilität auf gekälkten Unterlagen aus.

Die Anwendung der erfindungsgemäßen Wirkstoffe geschieht im Veterinärsektor in bekannter Weise, wie durch orale Anwendung in Form von beispielsweise Tabletten, Kapseln, Tränken, Granulaten, durch dermale Anwendung in Form beispielsweise des Tauchens (Dippen), Sprühens (Sprayen), Aufgießens (pour-on and spot-on) und des Einpuderns sowie durch parenterale Anwendung in Form beispielsweise der Injektion.

Die erfindungsgemäßen Wirkstoffe können auch in Mischung mit anderen bekannten Wirkstoffen wie Phosphorsäureestern, Carbonsäureestern, einschließlich der natürlichen und synthetischen Pyrethroide, Carbamaten oder Halogenalkanen angewendet werden.

Die erfindungsgemäßen Wirkstoffe weinen auch eine starke fungitoxische und bakteriotoxische Wirkung auf. Sie schädigen Kulturpflanzen in den zur Bekämpfung von Pilzen und Bakterien notwendigen Konzentrationen nicht. Aus dieaon Grünen sind sie auch für den Gebrauch als Pflanzenschutzmittel zur Bekämpfung von Pilzen und Bakterien geeignet. Funqitoxische Mittel im Pflanzenschutz werden eingesetzt zur Bekämpfung von Plasmodiophoromycetes, Oomycetes, Chytridiomycetes, Zygomycetes, Ascomycetes, Basidiomycetes, Deuteromycetes.

Die erfindungsgemäßen Wirkstoffe haben ein breites Wirkungsspektrum und können angewandt werden gegen parasitäre Pilze, die oberirdische Pflanzenteile befallen oder die Pflanzen vom Boden her angreifen, sowie gegen samenübertragbare Krankheitserreger.

Bei den folgenden Beispielen zur entwicklungshemmenden Wirkung der Wirkstoffe werden während der gesamten angegebenen Entwicklung der Testtiere die morphologischen Veränderungen, wie zur Hälfte verpuppte Tiere, unvollständig geschlüpfte Larven oder Raupen, defekte Flügel, pupale Kutikula bei Imagines etc., als Mißbildungen gewertet. Die Summe der morphologischen Mißbildungen, zusammen mit den während des Häutungsgeschehens oder der Metamorphose abgetöteten Tiere, wird in Prozent der Gesamtzahl der Versuchstiere bestimmt.

### Beispiel

Entwicklungshemmende Wirkung/Fraßtest

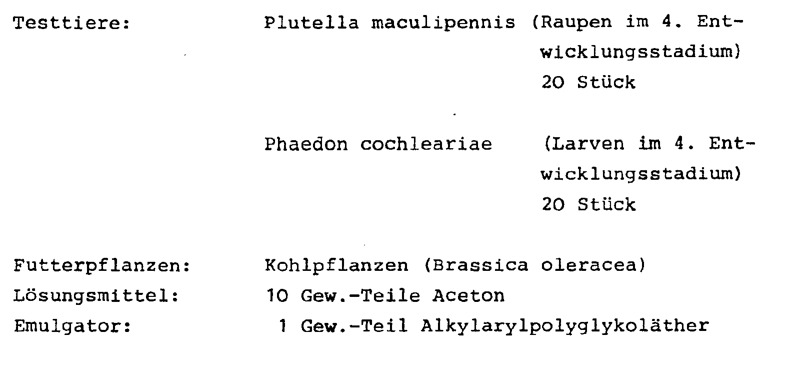

Zur Herstellung einer zweckmäßigen Wirkstoffzubereitung vermischt man 2 Gew.-Teile Wirkstoff mit der angegebenen Menge Lösungsmittel, Emulgator und soviel Wasser, daß eine 1 %ige Mischung entsteht, die mit Wasser auf die gewünschte Konzentration verdünnt wird.

Die Testtiere werden mit Blättern der Futterpflanzen, die mit einem gleichmäßigen Spritzbelag der Wirkstoffmischung so überzogen werden, daß die gewählte Wirkstoffkonzentration pro Flächeneinheit auf den Blättern erreicht wird, bis zur Entwicklung der Imago gefüttert.

Zur Kontrolle werden nur mit Lösungsmittel- und Emulgator-Wassergemisch der gewählten Konzentrationen überzogene Blätter verfüttert.

Die Ergebnisse gehen aus der folgenden Tabelle hervor:

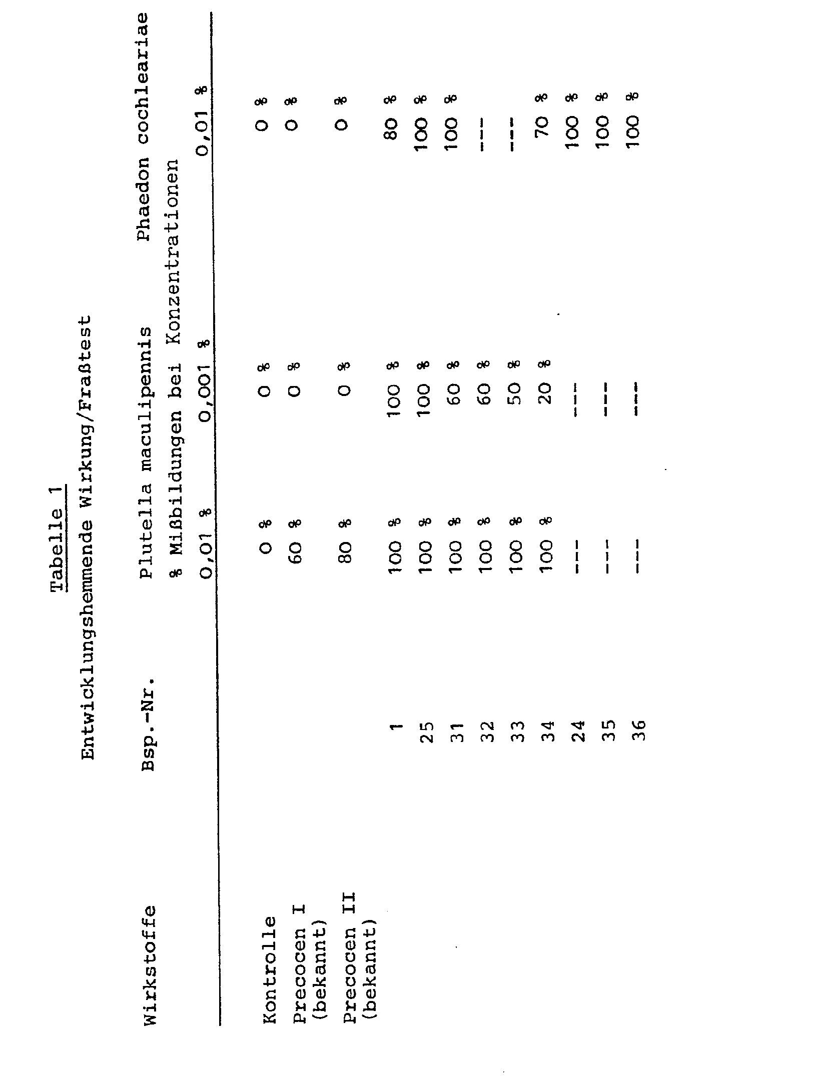

### Beispiel 2

Entwicklungshemmende Wirkung/Laphygma - Eier-Test

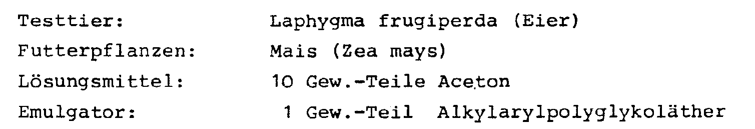

Zur Herstellung einer zweckmäßigen Wirkstoffzubereitung vermischt man 2 Gew.-Teile Wirkstoff mit der angegebenen Menge Lösungsmittel, Emulgator und soviel Wasser, daß eine 1 %ige Mischung entsteht, die mit Wasser auf die gewünschte Konzentration verdünnt wird.

Eigelege aus 30 Eiern auf Filterpapier werden mit 1 ml Wirkstofflösung der gewählten Konzentration angefeuchtet und in Kunststoffdosen bis zum Schlupf der Junglarven beobachtet. Die Junglarven werden mit Maisblättern gefüttert, die mit der Wirkstofflösung der gewählten Konzentration am gleichen Tag wie die Eier besprüht wurden. Die Entwicklung der Versuchstiere wird bis zur Larve des 3. Stadiums beobachtet.

Zur Kontrolle werden Eigeleqe in gleicher Weise mit Lösungsmittel- und Emulgator-Wassergemisch der gewählten Konzentration behandelt und mit entsprechend behandelten Maisblättern gefüttert.

Die Ergebnisse gehen aus der nachfolgenden Tabelle hervor:

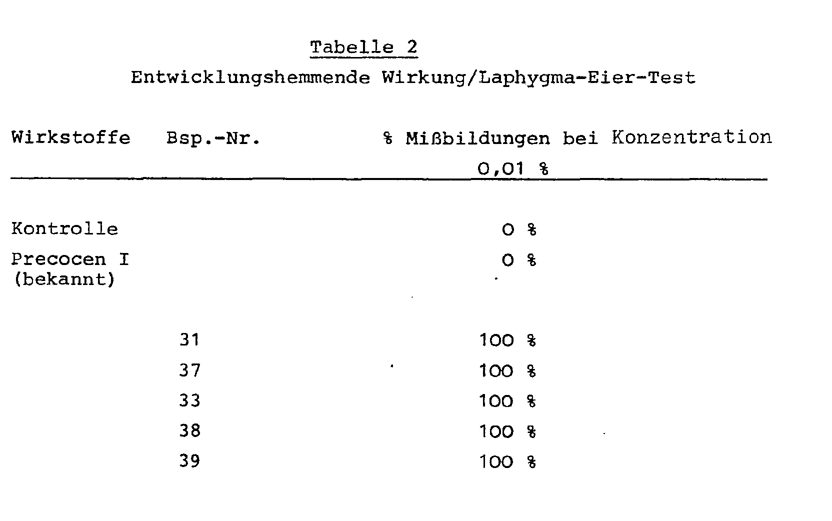

Im folgenden sei die Herstellung einiger der erfindungsgemäßen Wirkstoffe illustriert.

### Beispiel 1

### Verfahren A:

Ein Gemisch von 600 g o-Hydroxy-acetophenon, 1 1 Methanol und 630 g 1-N-Pyrrolidinylcyclopenten wird 2 Stunden auf Rückflußtemperatur erwärmt und dann eingeengt. Der Rückstand liefert bei der fraktionierten Destillation neben einem Vorlauf 770 g (86 % der theoretischen Ausbeute) 2,2-Tetramethylenchromanon-(4); Siedepunkt 100 - 105⁰C/0,05 Torr.

### Verfahren B:

Ein Gemisch von 680 g o-Hydroxy-acetophenon, 1 1/2 1 Toluol und 550 g Cyclopentanon wird mit 100 g Pyrrolidin versetzt, 1 Tag bei 25° stehen gelassen und anschließend 5 Stunden an einem Wasserabscheider erhitzt. Nach dem Abkühlen schüttelt man die organische Phase mit 250 ml 2n-NaOH, 700 ml 2n-HCl und 500 ml Wasser aus, trocknet die Toluollösung über Natriumsulfat, engt ein und destilliert. Bei einer Siedetemperatur von 110 - 120°C bei 0,1 Torr erhält man 860 g (85 % der theoretischen Ausbeute) 2,2-Tetramethylenchromanon-(4).

Die nach beiden Varianten erhaltenen 2,2-Tetramethylenchromanone-(4) sind identisch; sie zeigen im Kernresonanzspektrum die erwarteten Signale und im Infrarotspektrum eine starke Bande bei 1680 - 1690 cm.

Die in der folgenden Tabelle angeführten Chromanone-(4) sind nach Variante A oder B hergestellt.

Nach Variante A wird eine o-Hydroxy-arylcarbonylverbindung der Formel 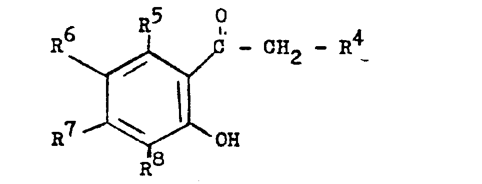 mit einem Enamin der Formel 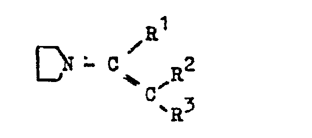 umgesetzt. In der folgenden Tabelle sind die jeweiligen Substituenten angegeben.

Nach Variante B wird eine o-Hydroxy-arylcarbonylverbindung der Formel 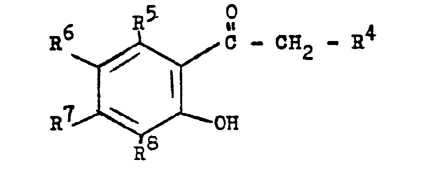 mit einer Carbonylverbindung der Formel 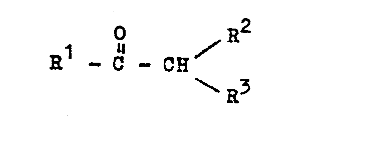 in Gegenwart von Pyrrolidin umgesetzt. In der folgenden Tabelle sind die jeweiligen Substituenten angegeben. Außerdem ist in der Tabelle die molare Menge des Pyrrolidins bezogen auf die o-Hydroxy-arylcarbonylverbindung angeführt.

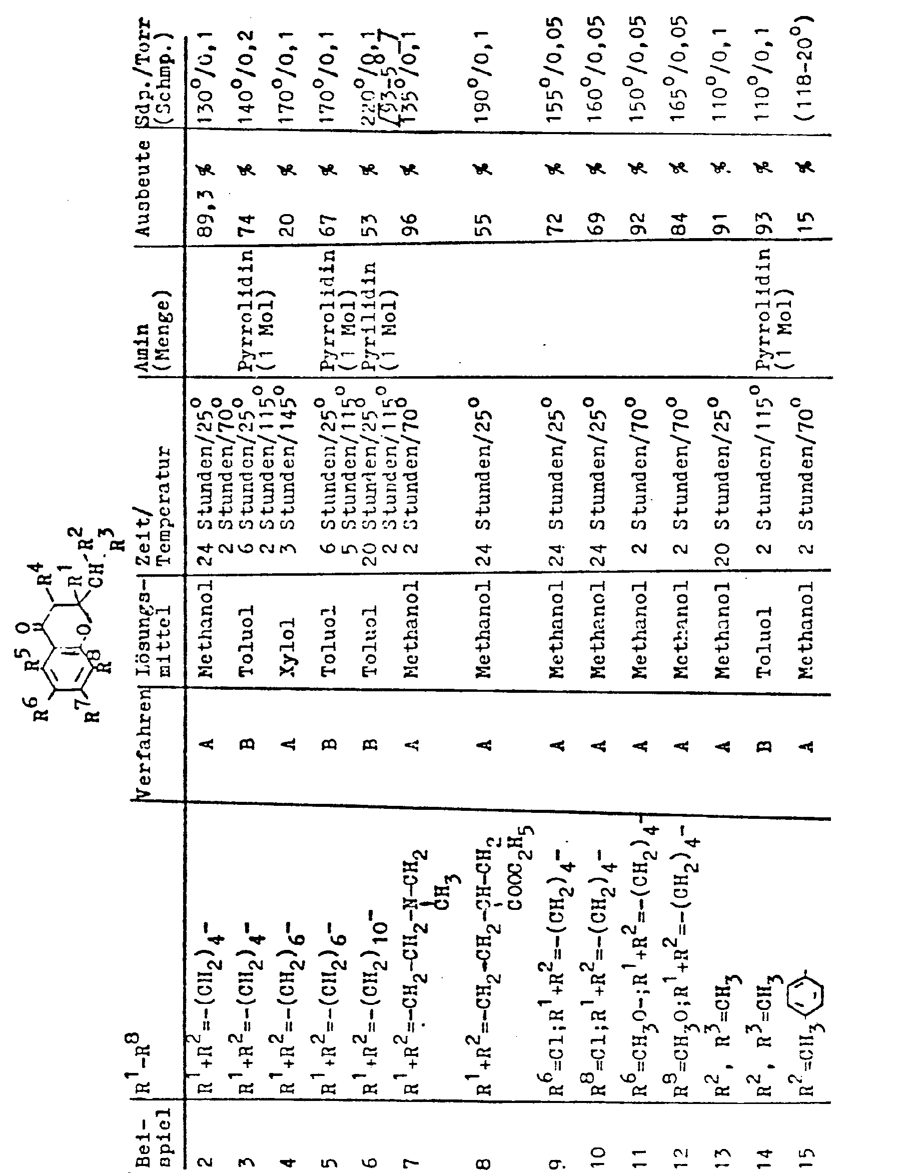

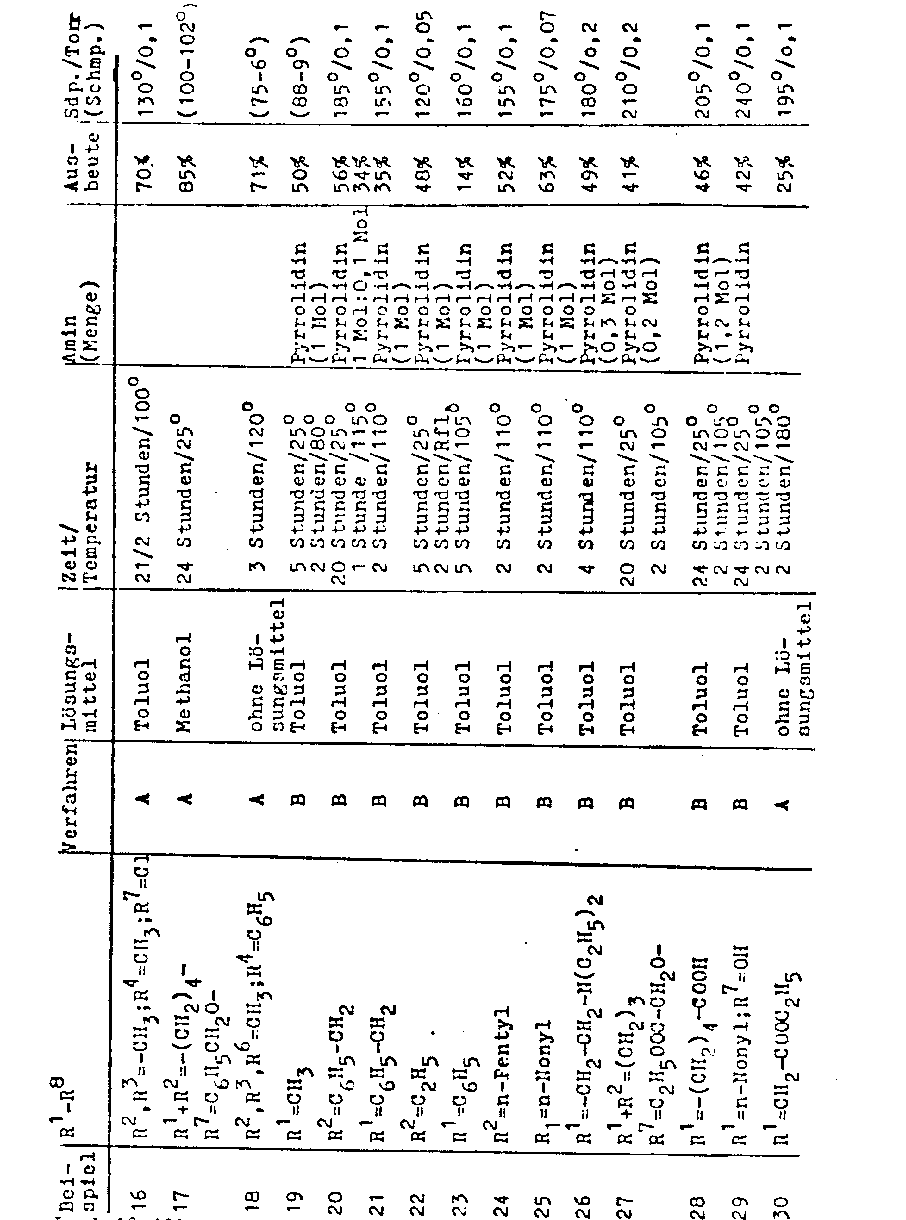

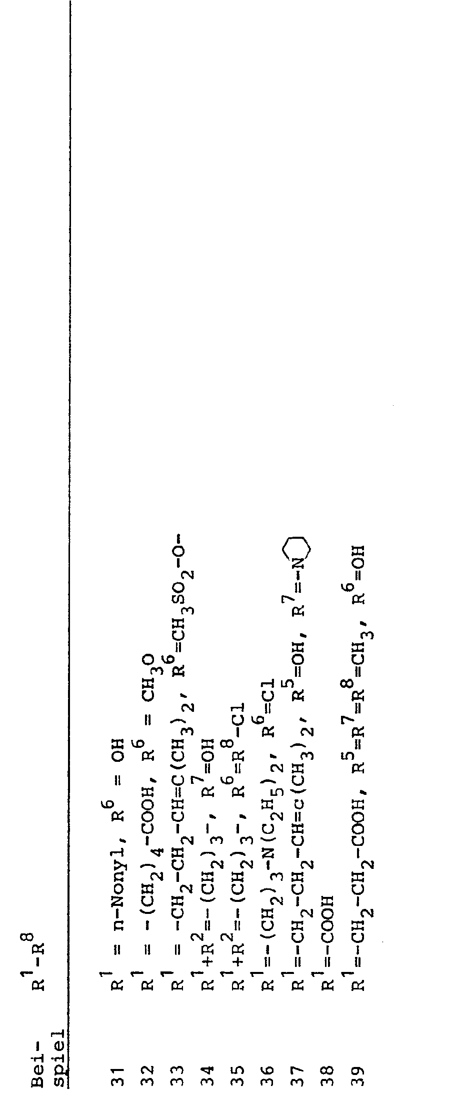

## Patentansprüche

1. Insektizide und akarizide Mittel, gekennzeichnet durch einen Gehalt an mindestens einem Chromanon-(4) der allgemeinen Formel (I) 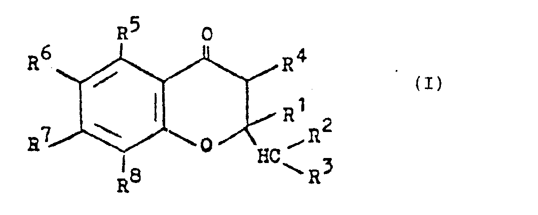 worin
_{R}¹ bis _{R}⁴ gleich oder verschieden sind und für Wasserstoff, gegebenenfalls substituiertes Alkyl, Alkenyl, Cycloalkyl, Aryl, Aralkyl, Alkoxycarbonyl, Carboxyl und Aminoalkyl stehen,
_{R}² zusätzlich für Amino und Dialkylamino stehen kann und
R und R² gemeinsam mit den angrenzenden C-Atomen einen carbocyclischen oder heterocyclischen Ring bilden können und
R⁵ bis R⁸ gleich oder verschieden sind und für Wasserstoff, Halogen, Hydroxy, Nitro, Cyan, Carboxy, gegebenenfalls substituiertes Alkyl, Alkenyl, Cycloalkyl, Aryl, Aralkyl, Alkoxy, Aralkoxy, Aryloxy, Alkoxycarbonyl, Amino, Alkylamino, Dialkylamino oder Acylamino stehen.

2. Verwendung von Chromanonen-(4) der allgemeinen Formel (I), in Anspruch 1 zur Bekämpfung von Insekten oder Spinnentieren.

3. Verfahren zur Bekämpfung von Insekten oder Spinnentieren, dadurch gekennzeichnet, daß man Chromanone-(4) der allgemeinen Formel (I) in Anspruch 1 auf Insekten oder Spinnentiere und/oder ihren Lebensraum einwirken läßt.

4. Verfahren zur Herstellung insektizider oder akarizider Mittel, dadurch gekennzeichnet, daß man Chromanone-(4) der allgemeinen Formel (I) in Anspruch 1 mit Streckmitteln und/oder oberflächenaktiven Stoffen vermischt.
